# EUROPEAN PATENT SPECIFICATION

(11) **EP 2 618 852 B1**
(45) Date of publication and mention of the grant of the patent: **11.06.2014**
(21) Application number: 11773828.6
(22) Date of filing: 07.09.2011
(51) Int. Cl.: A61L 2/18, A61L 2/22, B08B 3/02, B60S 3/00

(54) **PLANT FOR WASHING TROLLEYED OBJECTS**
ANLAGE ZUR REINIGUNG VON AUF WAGEN TRANSPORTIERTEN OBJEKTEN
UNITÉ DESTINÉE AU LAVAGE D'OBJETS DÉPLACÉS SUR UN CHARIOT

(30) Priority: 20.09.2010 IT UD20100169
(43) Date of publication of application: 31.07.2013
(73) Proprietor: Steelco Spa, 31039 Riese Pio X (IT)
(72) Inventor: ZARDINI, Fabio, I-31033 Castelfranco Veneto (IT)
(74) Representative: Petraz, Gilberto Luigi
(86) International application number: PCT/IB2011/002086
(87) International publication number: WO 2012/038792

(56) References cited:
- WO-A1-2010/049402
- DE-A1- 19 611 391
- US-A- 5 993 739
- US-A1- 2010 122 717
- US-B1- 6 244 278

## Description

### FIELD OF THE INVENTION

The present invention concerns a plant for washing trolleyed objects, for example trolleys, beds, gurneys or other apparatus provided with wheels, in a washing chamber, of the type used in hospital environments. By washing chambers we mean, here and in hereafter, a chamber in which the trolleyed objects can be subjected to at least one of the following treatments: pre-washing in cold and/or hot water, washing in hot water advantageously with detergents, rinsing, chemical-thermal disinfection and thermo-disinfection, with a possible final drying.

### BACKGROUND OF THE INVENTION

Washing chambers are known, for example those used in hospital environments or in laboratories, to wash re-usable apparatus such as trolleys, beds, gurneys, stretchers or similar trolleyed objects provided with wheels.

Known washing chambers are usually provided with an entrance aperture, through which the trolleyed objects to be washed are introduced when they are soiled, with and a loading plane, for example a grid, on which the soiled objects are pushed.

Known washing chambers are also provided with nozzles to deliver jets of cold and/or hot water which, with the help of suitable detergents, allow for example to wash and disinfect the trolleyed objects disposed on said plane. In such washing chambers it is also possible to carry out thermo-disinfection by means of super-heated water.

The washed trolleyed objects are then removed through an exit aperture, which normally faces toward a zone separate from the zone where the soiled apparatus is introduced, so as to avoid contact or contamination between soiled objects and those which have been washed.

Document US-A-2010/122717 shows an example of a washing machine of the known type for trolleyed objects, by means of which the trolleyed objects can be loaded, washed and unloaded automatically.

Moreover, also the international application WO-A-2010/049402 in the name of the Applicant describes an apparatus for washing trolleyed objects provided with insertion and extraction devices for the objects treated in a washing chamber.

In particular, the extraction operations of the washed objects are carried out in an automated way, in order to reduce to a minimum any manual contact by the operators, once the bed or other trolleyed object has been washed.

In some known automated extraction apparatuses of washed trolleyed objects a slider is provided, disposed in a first position of use inside the washing chamber, and in cooperation with which the trolleyed objects to be washed, or containing the objects to be washed, are disposed inside the washing chamber.

According to this known solution, the automatic extraction device, working under traction, hooks the slider to extract it from the washing chamber and take it to the loading zone and, in association with the hooking action, an interference mechanism is activated which cooperates with the wheels of the trolleyed object, constraining it to the slider and thus determining the extraction of the trolleyed objects from the washing chamber together with the slider.

It is also known that trolleyed objects, depending on the make, the type of object, their functionality or other, have wheels with a different pitch, wheelbase and distance in width, and also different sizes.

Therefore, an apparatus which provides cooperation with said wheels to carry out the extraction from the washing chamber must necessarily provide specific equipment of its interference mechanisms, depending on the actual dimensions and wheelbase.

Operationally there is the need to introduce into the washing chamber, in sequence, trolleyed objects which are different with respect to each other.

This operational procedure contrasts with the need for specific equipment depending on the specific trolleyed objects which we have with known solutions, causing increased operating times and costs.

Purpose of the present invention is to achieve a plant to wash trolleyed objects in a washing chamber which is of the universal type, that is, which allows to automate the extraction operations substantially of any type of trolleyed object, even loaded in sequence one different from the other into the washing chamber.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

In accordance with the above purpose, a plant according to the present invention comprises a washing chamber, inside which trolleyed objects can be washed, such as trolleys, beds, gurneys or other objects which have wheels, and objects in general contained in the suitable sliders.

According to the present invention, the plant comprises an automatic extraction device working under traction to extract the trolleyed objects from inside the washing chamber to a loading zone outside the washing chamber.

According to the present invention, the automatic extraction device comprises an extraction arm provided with attachment means able to cooperate from above with the head wall, and having at least a first position in which the attachment means are inside the washing chamber and cooperate from above, at least temporarily, with the head wall, and a second position, in which said attachment means are outside the washing chamber and extract, under traction, said trolleyed object from the washing chamber.

According to one feature of the present invention, the extraction arm is movable in height by means of relative actuator means, so as to be able to position the relative attachment means at a height from the ground coordinated with the height of the head wall of the trolleyed object.

In this way, the advantage is obtained that objects of various height can be treated by means of the same extraction device, by selectively adjusting the operative height of the extraction arm.

According to one form of embodiment, the extraction arm is assembled on a movement mechanism able to move the extraction arm at least between the first position and the second position.

In one form of embodiment, the extraction device comprises at least an unloading structure outside the washing chamber.

In a variant, the movement mechanism is assembled on an upper wall of the unloading structure.

Thanks to the configuration of the present invention the automatic extraction device, working under traction during the extraction step, attaches the trolleyed object by one of its head walls to extract it from the washing chamber and take it into correspondence to the unloading structure.

With the present invention, by taking the attachment means into cooperation from above with a head wall of the trolleyed object, and not with the wheels of the latter as occurs in the known state of the art, the attachment drawbacks connected to the structural lack of uniformity of the wheels of the different trolleyed objects are reduced to a minimum.

Irrespective of the producer, the type and its functionality, any trolleyed object comparable to a bed, a gurney, a stretcher, a basket or others, provides a head wall, such as a headboard, a back or similar, with a substantially common or standardized shape and size, and in any case which can be freely accessed from above and with which the attachment means can cooperate in a substantially universal manner.

Therefore the solution according to the present invention considerably simplifies the automatic extraction operations of the trolleyed object and substantially eliminates the need for equipment in the plant depending on the type of trolleyed object to be washed, thus reducing operating times and costs and increasing the productivity of the plant itself, and furthermore the operator does not need to go into the chamber and so avoids finding himself in an unsuitable environment.

In one form of embodiment, in which the trolleyed object is a hospital bed, the attachment means cooperate with the upper edge of the headboard of the hospital bed in order to move it, under traction, outside the washing chamber.

According to a variant, the plant comprises sensor means disposed at the entrance to the washing chamber and able to detect the height from the ground at least of the head wall of the trolleyed object so that the extraction arm positions the relative attachment means at a height from the ground coordinated to the height measured of the head wall of the trolleyed object.

According to a further variant the automatic extraction device comprises two extraction arms disposed specular on the width of the unloading structure, so as to attach to two points of the head wall of the trolleyed object and stabilize the traction conditions thereof during its extraction from the washing chamber.

According to a variant, the attachment means comprise a hook element conformed to be constrained on a typical tubular element of a head wall or headboard of a trolleyed object or hospital bed.

According to another variant, the attachment means comprise a blade element conformed to rest on an internal surface of a panel of the head wall of the trolleyed object.

According to another variant the attachment means comprise a magnetic element able to contact an external surface of a panel of the head wall of the trolleyed object.

According to another variant the attachment means comprise a sucker element able to be constrained to an external surface of a panel of the head wall of the trolleyed object.

According to another variant the attachment means are able to be selectively constrained to a terminal of the extraction arm, for example by temporary constraining means such as screws, magnetic, bayonet, snap-in or other.

According to a variant, end-of-travel means are provided inside the washing chamber in order to detect the correct position of the trolleyed object inside the washing chamber so as to dispose it in cooperation with the attachment means of the extraction arm.

### BRIEF DESCRIPTION OF THE DRAWINGS

These and other characteristics of the present invention will become apparent from the following description of a preferential form of embodiment, given as a non-restrictive example with reference to the attached drawings wherein:
- Fig. 1 shows a lateral view of a plant to wash trolleyed object according to the present invention;
- Fig. 2 shows a front view of the washing plant in fig. 1.

To facilitate comprehension, the same reference numbers have been used, where possible, to identify identical common elements in the drawings. It is understood that elements and characteristics of one form of embodiment can conveniently be incorporated into other forms of embodiment without further clarifications.

### DETAILED DESCRIPTION OF A PREFERENTIAL FORM OF

### EMBODIMENT

With reference to the attached drawings, a plant 10 to wash trolleyed objects is shown, in this case but not only, a hospital bed 11 which has at least a headboard 11a, in this case tubular, and a plurality of wheels 11b, by means of which it slides on the ground.

The plant 10 according to the present invention comprises a washing chamber 12 and an automatic extraction device 13 disposed outside the extraction chamber 12.

The washing chamber 12 typically comprises an entrance opening 14 through which the beds 11 to be washed are introduced, an exit opening 18 through which the washed beds 11 are extracted, and a plurality of nozzles, of the known type and not shown here, which are disposed inside the washing chamber 12 and are suitable to carry out the various cycles of rinsing, washing with disinfectant, drying and/or others as provided.

The automatic extraction device 13 works under traction on the bed 11, to extract it from the washing chamber 12 at the end of the washing cycles, and move it to a discharge zone outside the washing chamber 12.

In particular the automatic extraction device 13 comprises a movement mechanism 16 and an extraction arm 17 assembled on the movement mechanism 16.

In this case the automatic extraction device 13 also comprises an unloading structure 15 defining the outside unloading zone, on which the movement mechanism 16 is assembled. The unloading structure 15 is for example made by means of a frame defining at least an upper wall 19, or in any case a zone which, in use, is above the bed 11 extracted from the washing chamber 12. In this case, the movement mechanism 16 is assembled on the upper wall 19.

The extraction arm 17 is provided with an attachment terminal 22 to cooperate from above with the headboard 11a.

The movement mechanism 16 is able to move the extraction arm 17 between at least a first position, in which the attachment terminal 22 is inside the washing chamber 12 and cooperates from above with the headboard 11a, and a second position in which the attachment terminal 22 is outside the washing chamber 12 in order to extract the bed 11, under traction, from the washing chamber 12.

The extraction arm 17 is also selectively mobile so that its position in height can be adjusted, by means of a relative actuator member 23, in a coordinated manner with the height of the bed 11, in particular of the headboard 11a or other object to be treated.

In this case the movement mechanism 16 comprises a pair of tracks 20 attached longitudinally to the upper wall 19 of the unloading structure 15 and a motorized slider 12, assembled sliding on the tracks 20, so as to selectively slide along them.

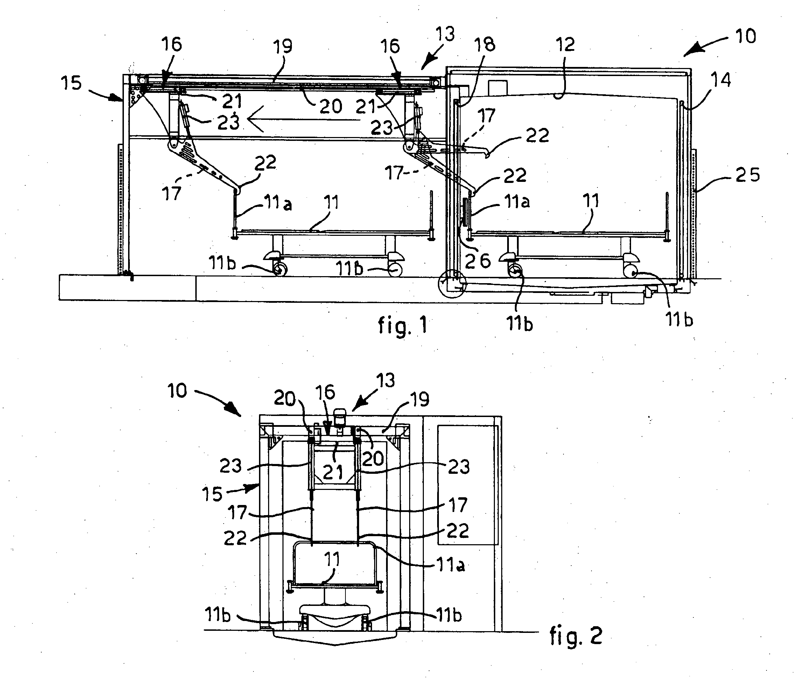

As shown in the example in fig. 2, two extraction arms 17 are provided, each of which is hinged to a slider 21 and comprises a corresponding attachment terminal 22. In this way, each extraction arm 17 is moved by the slider 21 from a first attachment position, in which the attachment terminal 22 is constrained to the headboard 11 a of the bed 11 inside the washing chamber 12, and a second discharge position in which the extraction arm 17 and the bed 11 constrained to it by means of the attachment terminal 22 are in the unloading zone in correspondence to the unloading structure 15.

In particular, the actuator member 23, which determines the rotation movement of the relative extraction arm 17 so as to selectively raise or lower the attachment terminal 22, is provided between the slider 21 and each extraction arm 17.

Each attachment terminal 22 is, in this case, conformed as a hook, so as to be attached by same-shape coupling to the tubular profile of the headboard 11a, thus constraining the bed to the extraction arm 17.

The plant 10 according to the present invention also comprises a height sensor 25, for example a photoelectric cell, disposed in correspondence to the entrance opening 14 of the washing chamber 12, and able to detect the maximum height of the headboard 11a of the bed 11 from the ground.

This value of the height as measured is sent to the automatic extraction device 13 so that the extraction arm 17 is rotated by the actuator member 23, so as to bring the attachment terminal 22 to an operating height coordinated to the height measured by the height sensor 25.

Moreover, the plant 10 according to the present invention comprises an end-of-travel sensor 26 disposed in proximity to the exit opening 18 and able to detect a determinate position of the bed 11 inside the washing chamber 12.

The position of the bed 11 detected by the end-of-travel sensor 26 corresponds to an ideal positioning of the headboard 11a of the bed 11 so that there is optimal attachment cooperation between the attachment terminal 22 and the headboard 11a.

The end-of-travel sensor can be of the mechanic type, for example like a little gate, or optical, magnetic, or any other type.

In this way, depending on the height detected by the height sensor 25 and on the position detected by the end-of-travel sensor 26, the slider 21, in cooperation with the actuator member 23, moves the extraction arm 17 and its attachment terminal 22, to move them to the first attachment position.

At this point, the slider 21 moves the extraction arm 17 to take it into its second extraction position completely outside the washing chamber 12.

In this position the actuator member 23 raises the extraction arm 17 so as to release the attachment terminal 22 from the tubular element of the headboard 11a of the bed 11.

It is clear that modifications and/or additions of parts may be made to the plant 10 as described heretofore, without departing from the field and scope of the present invention. It is also clear that, although the present invention has been described with reference to some specific examples, a person of skill in the art shall certainly be able to achieve many other equivalent forms of plant to wash trolleyed objects, having the characteristics as set forth in the claims and hence all coming within the field of protection defined thereby.

## Claims

1. Plant for washing trolleyed objects (11) provided with at least a head wall (11a), comprising at least a washing chamber (12) to wash said trolleyed objects (11), and an extraction device (13) working under traction, and able to extract said trolleyed objects (11) from inside said washing chamber (12) to an unloading zone outside said washing chamber (12), said extraction device (13) comprising an extraction arm (17) provided with attachment means (22) able to cooperate from above with said head wall (11a), and having at least a first position in which the attachment means (22) are inside said washing chamber (12) and cooperate from above with said head wall (11a), and a second position, in which said attachment means (22) are outside said washing chamber (12) and carry out the extraction, under traction, of said trolleyed object (11) from said washing chamber (12), **characterized in that** said extraction arm (17) is movable in height by means of relative actuator means (23), so as to be able to position the relative attachment means (22) at a height from the ground coordinated with the height of the head wall (11a) of the trolleyed object (11).

2. Plant as in claim 1, **characterized in that** said extraction arm (17) is assembled on a movement mechanism (16) suitable to move said extraction arm (17) at least between said first position and said second position.

3. Plant as in claim 1 or 2, **characterized in that** said extraction device (13) comprises at least an unloading structure (15) defining said unloading zone.

4. Plant as in claim 2 and 3, **characterized in that** said movement mechanism (16) is assembled on an upper wall (19) of said unloading structure (15).

5. Plant as in any claim hereinbefore, **characterized in that** it comprises sensor means (25) disposed at the entrance to the washing chamber (12) and able to detect the height from the ground at least of the head wall (11a) of the trolleyed object (11).

6. Plant as in claim 5, **characterized in that** the actuator means (23) move the extraction arm (17) so as to position it with the relative attachment means (22) at a height from the ground coordinated with the height of the head wall (11a) of the trolleyed object (11) measured by said sensor means (25).

7. Plant as in any claim hereinbefore, **characterized in that** the extraction device (13) comprises two extraction arms (17) disposed specular on the width of the unloading structure (15).

8. Plant as in any claim hereinbefore, **characterized in that** the attachment means (22) comprise a hook element conformed to be constrained to a tubular element of the head wall (11a) of the trolleyed object (11).

9. Plant as in any claim from 1 to 7, **characterized in that** the attachment means (22) comprise a blade element conformed to rest on an internal surface of a panel of the head wall (11a) of the trolleyed object (11).

10. Plant as in any claim from 1 to 7, **characterized in that** the attachment means (22) comprise a magnetic element able to contact an external surface of a panel of the head wall (11a) of the trolleyed object (11).

11. Plant as in any claim from 1 to 7, **characterized in that** the attachment means (22) comprise a sucker element able to be constrained to an external surface of a panel of the head wall (11a) of the trolleyed object (11).

12. Plant as in any claim hereinbefore, **characterized in that** the attachment means (22) are selectively constrainable to a terminal of the extraction arm (17).

13. Plant as in any claim hereinbefore, **characterized in that** it comprises end-of-travel means (26) disposed inside the washing chamber (12) in order to detect the correct position of the trolleyed object (11) inside said washing chamber (12), so as to dispose it in cooperation with the attachment means (22) of the extraction arm (17).

## Patentansprüche

1. Anlage zum Waschen von mit Rollen versehenen Objekten (11), die mit wenigstens einer Stirnwand (11a) ausgestattet sind, aufweisend wenigstens eine Waschkammer (12), um die mit Rollen versehenen Objekte (11) zu waschen, und eine Extraktionsvorrichtung (13), die unter Kraftschluss arbeitet und die in der Lage ist, die mit Rollen versehenen Objekte (11) von einem Inneren der Waschkammer (12) zu einer Ausladezone außerhalb der Waschkammer (12) zu extrahieren, wobei die Extraktionsvorrichtung (13) einen Extrahierarm (17) aufweist, der mit Mitnehmermitteln (22) ausgestattet ist, die in der Lage sind, von oben her mit der Stirnwand (11a) zu kooperieren, und der wenigstens eine erste Position hat, in welcher die Mitnehmermittel (22) im Innern der Waschkammer (12) sind und von oben her mit der Stirnwand (11a) kooperieren, und eine zweite Position hat, in welcher die Mitnehmermittel (22) außerhalb der Waschkammer (12) sind und die Extraktion, unter Kraftschluss, des mit Rollen versehenen Objekts (11) aus der Waschkammer (12) durchführen, **dadurch gekennzeichnet, dass** der Extrahierarm (17) höhenbewegbar ist mittels Relativ-Aktuator-Mitteln (23), um in der Lage zu sein, die Relativ-Mitnehmermittel (22) in einer Höhe vom Boden koordiniert mit der Höhe der Stirnwand (11a) des mit Rollen versehenen Objekts (11) zu positionieren.

2. Anlage gemäß Anspruch 1, **dadurch gekennzeichnet, dass** der Extrahierarm (17) an einem Bewegungsmechanismus (16) anmontiert ist, der geeignet ist, den Extrahierarm (17) wenigstens zwischen der ersten Position und der zweiten Position zu bewegen.

3. Anlage gemäß Anspruch 1 oder 2, **dadurch gekennzeichnet, dass** die Extraktionsvorrichtung (13) aufweist wenigstens eine Ausladestruktur (15), welche die Ausladezone definiert.

4. Anlage gemäß Anspruch 2 und 3, **dadurch gekennzeichnet, dass** der Bewegungsmechanismus (16) an einer oberen Wand (19) der Ausladestruktur (15) montiert ist.

5. Anlage gemäß irgendeinem vorhergehenden Anspruch, **dadurch gekennzeichnet, dass** sie aufweist Sensormittel (25), die am Eingang zu der Waschkammer (12) angeordnet sind und in der Lage sind, die Höhe vom Boden aus zu wenigstens der Stirnwand (11a) des mit Rollen versehenen Objekts (11) zu erfassen.

6. Anlage gemäß Anspruch 5, **dadurch gekennzeichnet, dass** die Aktuator-Mittel (23) den Extrahierarm (17) bewegen, um ihn mit den Relativ-Mitnehmer-Mitteln (22) in einer Höhe vom Boden koordiniert mit der Höhe der Stirnwand (11a) des mit Rollen versehenen Objekts (11) zu positionieren, die von den Sensormitteln (25) gemessen ist.

7. Anlage gemäß irgendeinem vorhergehenden Anspruch, **dadurch gekennzeichnet, dass** die Extraktionsvorrichtung (13) zwei Extraktionsarme (17) aufweist, die spiegelbildlich auf der Breite der Auslagestruktur (15) angeordnet sind.

8. Anlage gemäß irgendeinem vorhergehenden Anspruch, **dadurch gekennzeichnet, dass** die Mitnehmermittel (22) ein Hakenelement aufweisen, das eingerichtet ist, um zu einem Rohrelement der Stirnwand (11a) gezwungen zu werden.

9. Anlage gemäß irgendeinem Anspruch von 1 bis 7, **dadurch gekennzeichnet, dass** die Mitnehmermittel (22) ein Klingenelement aufweisen, das eingerichtet ist, um sich an einer inneren Fläche eines Paneels der Stirnwand (11a) des mit Rollen versehenen Objekts (11) abzustützen.

10. Anlage gemäß irgendeinem Anspruch von 1 bis 7, **dadurch gekennzeichnet, dass** die Mitnehmermittel (22) ein magnetisches Element aufweisen, das in der Lage ist, eine externe Fläche eines Paneels der Stirnwand (11a) des mit Rollen versehenen Objekts (11) zu kontaktieren.

11. Anlage gemäß irgendeinem Anspruch von 1 bis 7, **dadurch gekennzeichnet, dass** die Mitnehmermittel (22) ein Saugelement aufweisen, das in der Lage ist, an eine äußere Fläche eines Paneels der Stirnwand (11a) des mit Rollen versehenen Objekts (11) gezwungen zu werden.

12. Anlage gemäß irgendeinem vorhergehenden Anspruch, **dadurch gekennzeichnet, dass** die Mitnehmermittel (22) selektiv zu einem Endpunkt des Extraktionsarms (17) zwingbar sind.

13. Anlage gemäß irgendeinem vorhergehenden Anspruch, **dadurch gekennzeichnet, dass** sie aufweist Bewegungswegende-Mittel (26), die im Innern der Waschkammer (12) angeordnet sind, um die korrekte Position des mit Rollen versehenen Objekts (11) im Innern der Waschkammer (12) zu erfassen, um es in Kooperation mit den Mitnehmermitteln (22) des Extraktionsarms (17) anzuordnen.

## Revendications

1. Unité destinée au lavage d'objets déplacés sur un chariot (11) pourvue d'au moins d'une paroi frontale (11a), comprenant au moins une chambre de lavage (12) pour laver lesdits objets déplacés sur un chariot (11), et un dispositif d'extraction (13) fonctionnant en traction, et en mesure d'extraire lesdits objets déplacés sur un chariot (11) de l'intérieur de ladite chambre de lavage (12) jusqu'à une zone de déchargement à l'extérieur de ladite chambre de lavage (12), ledit dispositif d'extraction (13) comprenant un bras d'extraction (17) muni de moyens d'accrochage (22) en mesure de coopérer par le haut avec ladite paroi frontale (11a), et ayant au moins une première position dans laquelle les moyens d'accrochage (22) sont à l'intérieur de ladite chambre de lavage (12) et coopèrent par le haut avec ladite paroi frontale (11a), et une seconde position, dans laquelle lesdits moyens d'accrochage (22) sont en dehors de ladite chambre de lavage (12) et effectuent l'extraction, sous traction, dudit objet déplacé sur un chariot (11) de ladite chambre de lavage (12), **caractérisée en ce que** ledit bras d'extraction (17) est mobile en hauteur par l'intermédiaire de moyens d'actionnement (23) relatifs, de manière à être en mesure de positionner des moyens d'accrochage (22) relatifs à une hauteur par rapport au sol coordonnée avec la hauteur de la paroi frontale (11a) de l'objet déplacé sur un chariot (11).

2. Unité selon la revendication 1, **caractérisée en ce que** ledit bras d'extraction (17) est monté sur un mécanisme de déplacement (16) adapté pour déplacer ledit bras d'extraction (17) au moins entre ladite première position et ladite seconde position.

3. Unité selon la revendication 1 ou la 2, **caractérisée en ce que** ledit dispositif d'extraction (13) comprend au moins une structure de déchargement (15) définissant ladite zone de déchargement.

4. Unité selon la revendication 2 ou 3, **caractérisée en ce que** ledit mécanisme de déplacement (16) est monté sur une paroi supérieure (19) de ladite structure de déchargement (15).

5. Unité selon l'une quelconque des revendications précédentes, **caractérisée en ce qu'**elle comprend des moyens de détection (25) disposés à l'entrée de la chambre de lavage (12) et capables de détecter la hauteur par rapport sol au moins de la paroi frontale (11a) de l'objet déplacé sur un chariot (11).

6. Unité selon la revendication 5, **caractérisée en ce que** les moyens d'actionnement (23) déplacent le bras d'extraction (17) de manière à positionner les moyens d'accrochage (22) relatifs à une hauteur par rapport du sol en coordonnée avec la hauteur de paroi frontale (11a) de l'objet déplacé sur un chariot (11) mesurée par ledit moyen de détection (25).

7. Unité selon l'une quelconque des revendications précédentes, **caractérisée en ce que** le dispositif d'extraction (13) comprend deux bras d'extraction (17) disposés de manière spéculaire sur la largeur de la structure de déchargement (15).

8. Unité selon l'une quelconque des revendications précédentes, **caractérisée en ce que** les moyens d'accrochage (22) comprennent un élément de crochet conformé pour être assujetti sur un élément tubulaire de la paroi frontale (11a) de l'objet déplacé sur un chariot (11).

9. Unité selon l'une quelconque des revendications 1 à 7, **caractérisée en ce que** les moyens d'accrochage (22) comprennent un élément de lame conformé pour reposer sur une surface interne d'un panneau de la paroi frontale (11) de l'objet déplacé sur un chariot (11).

10. Unité selon l'une quelconque des revendications 1 à 7, **caractérisée en ce que** les moyens d'accrochage (22) comprennent un élément magnétique capable de venir au contact d'une surface externe d'un panneau de la paroi frontale (11a) de l'objet déplacé sur un chariot (11).

11. Unité selon l'une quelconque des revendications 1 à 7, **caractérisée en ce que** les moyens d'accrochage (22) comprennent un élément d'aspiration capable d'être assujettis à une surface externe d'un panneau de la paroi frontale (11a) de l'objet déplacé sur un chariot (11).

12. Unité selon l'une quelconque des revendications précédentes, **caractérisée en ce que** les moyens d'accrochage (22) peuvent être sélectivement assujettis à une borne du bras d'extraction (17).

13. Unité selon l'une quelconque des revendications précédentes, **caractérisée en ce qu'**il comprend des moyens de fin de course (26) disposés à l'intérieur de la chambre de lavage (12) afin de détecter la position exacte de l'objet déplacé sur un chariot (11) à l'intérieur de ladite chambre de lavage (12), de manière à le disposer en coopération avec les moyens d'accrochage (22) du bras d'extraction (17).
